# EUROPÄISCHE PATENTSCHRIFT

(11) **EP 2 988 796 B1**
(45) Veröffentlichungstag und Bekanntmachung des Hinweises auf die Patenterteilung: **28.08.2019**
(21) Anmeldenummer: 14720928.2
(22) Anmeldetag: 16.04.2014
(51) Int. Cl.: A61M 1/10, A61M 1/14

(54) **KASSETTENMODUL MIT INTEGRIERTER ZENTRIFUGALPUMPENEINHEIT**
CASSETTE MODULE WITH INTEGRATED CENTRIFUGAL PUMP UNIT
MODULE À CASSETTE COMPRENANT UNE UNITÉ DE POMPE CENTRIFUGE INTÉGRÉE

(30) Priorität: 25.04.2013 DE 102013007190
(43) Veröffentlichungstag der Anmeldung: 02.03.2016
(73) Patentinhaber: Fresenius Medical Care Deutschland GmbH, 61352 Bad Homburg (DE)
(72) Erfinder: HEIDE, Alexander, 65817 Eppstein (DE); OERTER, Gökhan, 35789 Weilmünster (DE); WEIS, Manfred, 66606 St. Wendel (DE); WENKE, Marina, 66606 St. Wendel (DE); WIKTOR, Christoph, 63571 Gelnhausen (DE); WÄBER, Udo, 63071 Offenbach (DE)
(74) Vertreter: Herrmann, Uwe
(86) Internationale Anmeldenummer: PCT/EP2014/057683
(87) Internationale Veröffentlichungsnummer: WO 2014/173744

(56) Entgegenhaltungen:
- EP-A1- 1 930 034
- WO-A1-95/23627
- WO-A1-2006/120415
- WO-A1-2011/020600
- WO-A2-2006/135934
- DE-A1-102009 058 681
- US-A1- 2007 231 135
- US-A1- 2010 152 647

## Beschreibung

### Technisches Gebiet

Das Thema der Erfindung betrifft das Gebiet integrierter Kassettenmodule für den Einsatz in therapeutischen Verfahren der extrakorporalen Blutbehandlung, der Peritonealdialyse oder der Infusion.

Die Erfindung bezieht sich insbesondere auf therapeutische Behandlungen der Dialyse. Im Falle der extrakorporalen Blutbehandlung durch die Hämodialyse werden toxische Blutbestandteile über einen extrakorporalen Blutkreislauf an einer synthetischen Membran eines Dialysefilters abgetrennt.

Die Erfindung bezieht sich auch auf die Hämofiltration und Hämodiafiltration, deren Behandlungsverfahren mit den Verfahren der Hämodialyse verwand sind. Die vorliegende Erfindung ist für Verfahren der Hämofiltration und der Hämodiafiltration gleichermaßen anwendbar. Für weitere Erläuterung soll daher unter dem Begriff der Hämodialyse auch Hämofiltrations- und Hämodiafiltrationsverfahren verstanden werden.

Die Erfindung bezieht sich weiterhin auf die Peritonealdialyse. Bei der Peritonealdialyse wird das Peritoneum eines Patienten mit einer Dialysierflüssigkeit befüllt und toxische Metabolite werden über die Membran des Bauchfells in das Peritoneum geleitet. Die Dialysierflüssigkeit wird anschließend aus dem Peritoneum ausgeleitet.

Die Erfindung bezieht sich auch auf Blutbehandlungen der Transfusion, insbesondere der Zellseparation. In der Zellseparation wird Blut in einem Verarbeitungsschritt der Zentrifugation in einer Blutzentrifuge in seine Komponenten getrennt.

Die Erfindung bezieht sich auch auf Bereiche der Infusion, in der Behandlungsflüssigkeiten dem Patienten infundiert werden.

Die Verabreichung von Flüssigkeiten an einen Patienten in den genannten Behandlungen muss aus medizinischer Sicht kontrolliert und unter Sicherheitsvorkehrungen vor sich gehen. Wesentliche Aufgaben der Flüssigkeitsverarbeitung von zu verabreichenden Flüssigkeiten bestehen in der Temperierung von Flüssigkeiten, der Entgasung und Gasabscheidung, der Bilanzierung von entnommenen und verabreichten Flüssigkeiten, der Förderkontrolle entnommener und verabreichter Flüssigkeit, der Filtration sowie der Analyse der geförderten Flüssigkeiten.

Zur weiteren Begriffsvereinheitlichung werden Prozessschritte der eben genannten Art an behandlungsrelevanten Flüssigkeiten der extrakorporalen Blutbehandlung, der Peritonealdialyse oder der Infusion im Zusammenhang der vorliegenden Beschreibung der Erfindung als Flüssigkeitsverarbeitung bezeichnet.

Kassettenmodule werden in den genannten therapeutischen Verfahren verwendet, um behandlungsrelevante Flüssigkeiten, die einem Patienten verabreicht werden in diesem Sinne zu verarbeiten.

Das Ziel in derzeitiger Entwicklung von Kassettenmodulen ist es, zunehmend mehr Funktionen der Flüssigkeitsverarbeitung auf Kassettensystemen zu integrieren.

In derzeit üblichen therapeutischen Verfahren der genannten Art wird ein Kassettenmodul durch eine Maschineneinheit einer Behandlungsmaschine betrieben, die gezielt die Funktionseinheiten auf der Kassette zur Flüssigkeitsverarbeitung ansteuert. Die notwendigen Verarbeitungsschritte von Flüssigkeiten eines therapeutischen Verfahrens erfolgen dabei teilweise oder vorzugsweise ganz auf dem Kassettenmodul. Dies bietet den Vorteil, dass behandlungsrelevante Flüssigkeiten nicht, wie alternativ üblich, in Kontakt mit Teilen der Behandlungsmaschine kommen. Sofern die Kassettenmodule in ihrer Bauart als Einwegartikel ausgestaltet sind, können sie nach einer Behandlung entsorgt werden. Entsprechende Desinfektionsschritte können mit der Einmalverwendung der Kassetten eingespart werden, so dass auf zusätzliche Desinfektionseinrichtungen verzichtet werden kann. Die Maschinenseite ist damit konstruktiv weniger aufwendig ausgelegt. Ein hoher Integrationsgrad von Funktionseinheiten auf einer Kassette sichert zudem durch die kompakte Bauweise eine bedeutende Materialersparnis.

Kassettenmodule werden üblicherweise aus mehreren Komponenten zu einem Kassettenmodul aufgebaut. In der Regel wird ein Kassettenmodul aus einem oder mehreren biegesteifen Grundkörpern hergestellt. Als weitere Komponenten werden Folien und Schlauchstücke verwendet. Vorzugsweise werden die Komponenten aus Kunststoffmaterialien hergestellt, so dass sich dadurch die Möglichkeit bietet, Kassettenmodule wirtschaftlich in Massenfertigungsverfahren herstellen zu können. Insbesondere wird für die Herstellung der Kassettenmodule auf kostengünstige Extrusions- und Spritzgussverfahren zurückgegriffen. Ein wesentliches Erfordernis in der Entwicklung und Produktion von Kassettenmodulen ist die Möglichkeiten ein Kassettenmodul in möglichst wenigen einfachen Prozessschritten herstellen zu können. Mit diesem Gedanken verbunden ist letztendlich das Bestreben, die Herstellkosten in der Produktion der Kassettenmodule gering zu halten.

Kassettenmodule werden insbesondere auch mit integrierten Pumpenmittel hergestellt. Bekannte Kassettenmodule beinhalten Schlauchsegmente, die durch einen Pumpenaktor, z.B. dem Rotor einer Rollenpumpe oder den Stempeln einer Fingerpumpe, peristaltisch Flüssigkeit durch Kassette und Patientenschlauchabschnitte fördern.

Ein alternativer Pumpenmechanismus für extrakorporale Blutbehandlung und Infusionsbehandlungen wird auf Kassettenmodulen durch Zentrifugalpumpen bereitgestellt. Zu den Zentrifugalpumpen werden unter anderem auch die als Impellerpumpen bezeichneten Pumpen gezählt. Bei diesen Pumpen ist ein Rotor als Zentrifugalpumpenmittel in einem umgebenden Gehäuse aufgenommen. Flüssigkeiten werden durch das Gehäuse geleitet, indem der Rotor in Rotation versetzt wird. Solche Pumpen finden häufig in der Kardiotechnik und der Blutoxygenierung Verwendung. Nicht zuletzt aufgrund der Entwicklung neuer magnetischer Materialien mit hohen magnetischen Leistungseigenschaften können die Rotoren durch ein umgebendes äußeres Magnetfeld magnetisch berührungsfrei in den Pumpengehäusen gelagert werden. Als Pumpenaktor dient nun lediglich eine Einheit, die auf einer Maschinenseite zum Antrieb des Rotors ein Magnetfeld erzeugt. Werden Pumpenmittel und Pumpenaktor in magnetischem Eingriff gebracht, kann auf jegliche mechanische Ansteuerung des Rotors zu verzichten werden.

### Stand der Technik

Die DE 39 23 692 A1 zeigt eine Blutbehandlungseinheit zur Oxygenierung von Blut. In der Behandlungseinheit ist ein Rotor integriert, der Blut durch die Behandlungseinheit fördert.

Die WO 95/23627 A1 zeigt eine Kassette zur Förderung von Wundspülflüssigkeit. Zwischen zwei Plattenteilen der Kassette ist ein Fluidpfad für die Spülfluidflüssigkeit definiert, in dem ein Rotor zur Förderung von Spülflüssigkeit angeordnet ist. Der Rotor wird mechanisch über eine Welle, die mit einem Antriebsmotor in Verbindung steht, betrieben.

Die EP 1 909 864 A1 zeigt ein Kassettensystem, bei der zwischen zwei Kassettenplatten ein Rotor einer Impellerpumpe angeordnet ist. Der Rotor ist rein magnetisch gelagert und kann durch ein externes Magnetfeld, dass durch eine Kontrolleinheit bereitgestellt wird, angetrieben werden.

Die DE 10 2009 058681 A1, die US 2010/152647 A1 und die WO 2006/135934 A2 zeigen jeweils für sich genommen ein Kassettenmodul, das sämtliche Merkmale nach dem Oberbegriff des geltenden Anspruchs 1 aufweist.

Ausgehend vom relevanten Stand der Technik ist bisher keine Ausführung eines Kassettenmoduls mit einem Grundkörper bekannt, der vorbereitete Strukturen für die Aufnahme eines Zentrifugalpumpenmittels und die Führung von Flüssigkeitsströmen aufweist, und die Strukturen dabei so angeordnet und konstruiert sind, dass sie mit dem Grundkörper in einem einfachen Spritzgussprozess hergestellt werden können.

### Darstellung der Erfindung

Ein Problem in der Konstruktion und Herstellung eines Grundkörpers eines Kassettenmoduls mit einer Aufnahmeeinheit für ein Zentrifugalpumpenmittel und integrierten Flüssigkeitskanälen durch einen einfachen spritzgusstechnischen Prozessschritt ergibt sich aus der Bauart und der Strömungscharakteristik von Zentrifugalpumpen. Die Einströmung von Flüssigkeit in das Pumpengehäuse muss axial erfolgen, und zwar entlang der Rotationsachse des Rotors. Ein Ausströmen von durch den Rotor beschleunigter Flüssigkeit muss dagegen tangential zum rotierenden Rotor erfolgen, um eine maximale Strömungsenergie der Flüssigkeit ausnutzen zu können.

Dies erfordert, dass der Ein- und Austritt von Flüssigkeiten in das Pumpengehäuse auf unterschiedlichen Ebenen erfolgen muss. Für die Integration einer Zentrifugalpumpeneinheit auf einem Kassettenmodul stellt sich daraus folgend das Problem, die Zentrifugalpumpeneinheit so auf dem Grundkörper des Kassettenmoduls anzuordnen, dass die auf unterschiedlichen Niveaus liegenden Ein- und Austrittsöffnungen optimal mit auf dem Grundkörper integrierten Flüssigkeitskanälen in Verbindung gebracht werden können. Insbesondere bestand das Problem darin, eine Geometrie für ein Spritzgussteil eines Kassettenmodulgrundkörpers zu finden, dass die eben genannten Anforderungen erfüllt und das durch einen einfachen einstufigen Prozessschritt im Spritzguss hergestellt werden kann.

Erfindungsgemäß wird die Aufgabe durch ein Kassettenmodul mit den Merkmalen von Anspruch 1 gelöst.

Darüber hinaus bestand das Bedürfnis, eine Möglichkeit zu finden, mit der ein Kassettenmodul mit Flüssigkeitskanälen und einer Aufnahmeeinheit für ein Zentrifugalpumpenmittel sicher mit einer für den Betrieb des Kassettemoduls vorgesehenen Maschinenkonsole in Eingriff gebracht werden kann, so dass eine sichere Aktuierung maschinenseitig und eine Flüssigkeitsverarbeitung auf dem Kassettenmodul gewährleistet werden kann.

Diese Aufgabe wird durch eine Anordnung aus einem Kassettenmodul und einer Maschinenkonsole nach den Merkmalen von Anspruch 11 gelöst.

Eine weitere Aufgabe bestand darin, ein einfaches Spritzgussverfahren zu finden, aufgrund dessen ein Grundkörper eines Kassettenmoduls herstellbar ist, der eine Aufnahmeeinheit für ein Zentrifugalpumpenmittel beinhaltet und Flüssigkeitskanäle aufweist, die eine Flüssigkeitsverbindung zu der Aufnahmeeinheit herstellen.

Ein erfindungsgemäßes Kassettenmodul baut sich demnach, unter anderem, aus einem Grundkörper auf, der eine erste Seite und eine zweite Seite aufweist. Auf dem Grundkörper integriert befinden sich Flüssigkeitskanäle, die dafür vorgesehen sind, Blut oder andere Behandlungsflüssigkeiten zu verschiedenen Funktionseinrichtungen auf dem Kassettemodul zu leiten. Die Flüssigkeitskanäle können auf einer ersten Seite des Kassettenmoduls integriert sein, oder auf einer ersten und einer zweiten Seite des Kassettenmoduls. Weiterhin umfasst der Grundkörper eine Aufnahmeeinheit für ein Zentrifugalpumpenmittel. Insbesondere werden durch den Betrieb des Zentrifugalpumpenmittels Flüssigkeiten wie Blut oder Behandlungsflüssigkeiten, z.B. Dialysierflüssigkeiten, durch die Kassette gefördert. Die Aufnahmeeinheit ist in vorzugsweisen Ausführungen ein integraler Bestandteil des Grundkörpers.

Der Grundkörper bildet einen Teil eines Kassettenmoduls. Er kann insbesondere mit weiteren Komponenten verbunden werden, um ein betriebsbereites Kassettenmodul zu liefern. Es hat sich bewährt, dass Kassettenmodule in einer flachen Bauweise hergestellt werden, damit ein optimaler Eingriff von Kassettenmodul und Dialysemaschine entstehen kann.

Entsprechend kann ein Grundkörper eine flächige Konstruktion sein, dessen Seitenlängen ein Vielfaches der Höhe des Grundkörpers aufweist. Der Grundkörper zeigt in dieser Betrachtung eine erste Seite, die eine obere Seite darstellen kann, wenn der Grundkörper auf die Seite gelegt wird, die gegenüber der Höhe die langen Seitenlängen aufweist.

Auf einer ersten Seite, z.B. die obere Seite oder auf einer ersten Seite und einer zweiten Seite des Grundkörpers sind integrierte Flüssigkeitskanäle angeordnet. Unter integriert ist hierbei zu verstehen, dass die Kanäle und der Grundkörper einstückig hergestellt sind. Die Aufnahmeeinheit für ein Zentrifugalpumpenmittel ist auf einer zweiten Seite, z.B. der in der beschriebenen Betrachtung unteren Seite des Grundkörpers angeordnet.

Neben der bereits rein magnetischen Lagerung des Rotors ist es auch möglich, dass die Lagerung strömungsmechanisch und magnetisch erfolgt, oder dass die Lagerung teilweise durch eine mechanische Lagerung bewerkstelligt wird. Vorzuziehen ist im Hinblick auf die schonende Förderung von sensiblen Flüssigkeiten, wie z. B. Blut, dass der Rotor rein magnetisch gelagert wird. Eine strömungsmechanische Lagerung kann im Betrieb des Rotors sekundär auftreten, ist aber für die Lagerung bei Stillstand des Rotors nicht wirksam.

Die Aufnahmeeinheit umgreift den Rotor wenigstens teilweise und bildet mit diesem zusammen eine Pumpeneinheit. Gegebenenfalls können weitere Komponenten mit der Aufnahmeeinheit verbunden sein und den Rotor vollständig umschließen, so dass sich eine Pumpenkammer bildet, die im Betrieb von Flüssigkeit durchströmt wird. Vorzugsweise handelt es sich bei der Aufnahmeeinheit um einen Rohrstück oder Rohrstummel, der vom Radius so angepasst ist, dass er das Zentrifugalpumpenmittel, z. B. den Rotor einer Impellerpumpe, zumindestens teilweise umgibt.

Die Anordnung von Flüssigkeitskanälen und Aufnahmeeinheit in flüssigkeitsverbindender Konstruktion auf gegenüberliegenden Seiten des Grundkörpers ermöglicht eine einstückige Fertigung des Grundkörpers im Spritzgussverfahren.

Als Vorteil erweist es sich weiterhin, dass Flüssigkeitskanäle und Pumpeneinheit sehr kompakt auf dem Kassettenmodul hergestellt werden können. Die Förderwege, die Blut oder Behandlungsflüssigkeiten durchlaufen müssen, werden minimal gehalten, so dass Schädigungen an den Flüssigkeiten, z.B. durch Hämolyse oder Kontaminationen durch das Kassettenmaterial, gering gehalten werden können.

Die Möglichkeit den Grundkörper einstückig durch ein Spritzgussverfahren herstellen zu können, vermeidet Naht- und Kontaktstellen einer Mehrkomponentenherstellung. Dies begünstigt die Kompatibilität der Kassette gegenüber zu verarbeitetenden Flüssigkeiten wie, z.B. Blut oder anderen Behandlungsflüssigkeiten. Ein Kassettenmodul mit einem wie vorab beschriebenen hergestellten Grundkörper ist daher besonders dafür geeignet, in der Hämo- oder Peritonealdialyse verwendet zu werden.

In einer Ausführung kann der Grundkörper des Kassettenmoduls vorteilhaft so weitergebildet sein, dass Flüssigkeitskanäle auf einer ersten Seite des Grundkörpers Zu- und Ableitungen für Flüssigkeitsströme zur Aufnahmeeinheit des Pumpenrotors bilden. In diesem Fall weist der Grundkörper Durchgangspassagen auf, durch die das strömende Fluid von einer ersten Seite, auf denen die Flüssigkeitskanäle angeordnet sind, auf eine zweite Seite, auf der sich die Pumpeneinheit mit dem Zentrifugalpumpenmittel befindet, übertreten kann. Eine erste Durchtrittsöffnung ist vorteilhafterweise so angeordnet, dass die zuströmende Flüssigkeit in axialer Richtung auf den Rotor auftrifft. Eine zweite Durchtrittsöffnung ist so angeordnet, dass die tangential an der Aufnahmeeinheit des Zentrifugalpumpenmittels austretende Flüssigkeit auf die erste Seite geleitet wird.

Im Betrieb wird das Kassettenmodul mit der Seite, auf der sich die Pumpeneinheit, umfassend die Aufnahmeeinheit des Pumpenrotors, befindet, mit der Maschinenkonsole in Eingriff gebracht. Die Pumpeneinheit wird dann vorzugsweise in eine Vertiefung der Maschinenkonsole eingelegt. In dieser Anordnung ist es vorteilhaft, dass die flüssigkeitsführenden Kanäle auf der zweiten Seite angebracht sind.

Auf einer ersten Seite des Grundkörpers sind Flüssigkeitskanäle integriert, die mit der Pumpeneinheit auf der zweiten Seite in flüssigkeitsführender Verbindung stehen. Bevorzugt sind die Flüssigkeitskanäle dabei auf der ersten Seite auf der zur Maschinenkonsole abgewandten Seite angeordnet, wenn das Kassettenmodul betriebsbereit in die Behandlungsmaschine eingelegt ist. Eine Flüssigkeitsverbindung des tangentialen Auslasses der Pumpeneinheit auf der zweiten Seite des Grundkörpers und dem ableitenden Flüssigkeitskanal auf der ersten Seite wird durch eine Flüssigkeitsrampe ermöglicht. Die Flüssigkeitsrampe stellt einen schrägen ansteigenden Kanalabschnitt dar. Die Flüssigkeitsrampe stellt dabei einen Konstruktionsabschnitt dar, der ohne weiteres mit der weiteren Ausgestaltung des Grundkörpers in einem einstufigen Spritzgussprozess hergestellt werden kann.

In einer alternativen Ausgestaltung wird auf die Flüssigkeitsrampe verzichtet, da die Herstellung der Spritzgusswerkzeuge für eine derartige Geometrie, wie vorab beschrieben, sehr präzise ausgearbeitet werden muss. Es ist daher auch möglich, dass der tangentiale Auslass an der Aufnahmeeinheit für das Rotationspumpenmittel auf der zweiten Seite des Grundkörpers angebracht ist. Der tangentiale Auslass auf der zweiten Seite kann mit Schläuchen zur Weiterleitung der durchströmenden Flüssigkeit verbunden werden. Über den Schlauch können z.B. weitere flüssigkeitsführende Komponenten angeschlossen werden, die an dem Kassettenmodul separat angeschlossen sein können. Solche Komponenten können z.B. Dialysefilter, Tropfkammern, Gerinselfänger, Messschnittstellen sein. Der Grundkörper kann aber auch in dieser Anordnung einstückig durch einen einstufigen Spritzgussprozess hergestellt werden. In diesem Fall können auch auf der zweiten Seite des Grundkörpers Flüssigkeitskanäle angeordnet sein. Insbesondere ist der tangentiale Auslass an der Aufnahmeeinheit mit einem ableitenden Flüssigkeitskanal integral verbunden.

In einzelnen Fällen sind aber auch andere Konstruktionen denkbar, in denen der Grundkörper mit Flüssigkeitskanälen und die Aufnahmeeinheit für das Zentrifugalpumpenmittel zweiteilig aufgebaut sind. Wesentlich ist, dass Flüssigkeitskanäle auf einer ersten Seite des Grundkörpers angeordnet sind und sich Durchlässe im Grundkörper befinden, die die strömende Flüssigkeit auf die andere Seite des Grundkörpers passieren lassen. Die Aufnahmeeinheit kann ein separat hergestelltes Teil sein, das durch eine Klebeverbindung oder Schweißverbindung mit dem Grundkörper verbunden ist. Alternativ kann die Aufnahmeeinheit auch durch mechanische Befestigungsmittel mit dem Grundkörper verbunden werden.

Nach der Erfindung umfasst der Grundkörper eine Basisplatte, auf der die integralen Bestandteile angeordnet sind. Die Flüssigkeitskanäle werden dabei durch senkrechte Wandabschnitte gebildet, die senkrecht auf der Basisgrundplatte angeordnet sind. Unter senkrecht ist in diesem Zusammenhang keine strenge geometrische Anordnung von Basisplatte zur Kanalwandung zu verstehen. Für die Herstellung des Grundkörpers im Spritzgussprozess ist eine leichte Ausformschräge der Kanalwände notwendig, damit der spritzgegossene Grundkörper dem Spritzgusswerkzeug entnommen werden kann. Der Wandfuß, der direkt zur Basisplatte des Grundkörpers benachbart ist, kann daher im Querschnitt breiter ausgeführt sein, als die von der Basisplatte entfernten Wandabschnitte. Die Wand kann demnach z.B. im Querschnitt eine leichte konische oder konkave Form haben. Unter dem Begriff "senkrecht" wird daher in diesem Zusammenhang auch verstanden, dass lediglich die Hauptausrichtung der Wandebene senkrecht zur Hauptausrichtung der Basisplatte steht.

Die unter dieser Begrifflichkeit zu verstehenden senkrechten Wandabschnitte bilden eine seitliche Begrenzung der Flüssigkeitskanäle. Der Übergang von der Basisgrundplatte zu den senkrechten Wandabschnitten kann eckig oder verrundet ausgeführt sein, um beispielsweise die Kompatibilität zu sensiblen behandlungsrelevanten Flüssigkeiten, wie z.B. Blut, zu verbessern. Im Wesentlichen sind zwei Wandabschnitte mit einem Abstand parallel zueinander angeordnet, um einen Kanal zu bilden, so dass im Betrieb der Kassette eine Flüssigkeit wie Blut oder eine Behandlungsflüssigkeit zwischen den Wandabschnitten entlang des Kanals strömen kann. Zu diesem Zweck müssen die senkrechten Wandabschnitte überdeckt werden, so dass in Verbindung mit der Basisplatte den senkrechten Wandabschnitten und der Überdeckung ein geschlossener Kanal gebildet wird. In weiteren Abschnitten des Grundkörpers können die Wandabschnitte auch nicht parallel verlaufen. Insbesondere können die Wandabschnitte auch Teil einer funktionellen Untereinheit auf dem Grundkörper darstellen, z.B. einem Gerinselfänger für Blut oder eine Luftabscheidekammer, in der die Wandabschnitte nicht mehr parallel verlaufen.

Die Überdeckung kann durch eine biegesteife Platte erfolgen. Nach der Erfingung ist aber eine Überdeckung durch eine flexible Folie vorgesehen. Die Verwendung der flexiblen Folie für den Aufbau der Überdeckung hat den Vorteil, dass Toleranzen in der Fertigung des Grundkörpers leicht ausgeglichen werden können. Vorzugsweise sind die senkrechten Wandabschnitte so ausgebildet, dass alle Wandabschnitte die gleiche Höhe haben. Nach einem im Stand der Technik bekannten Schweißverfahren kann die überdeckende Folie oder Platte z.B. über eine Glasplatte auf den Grundkörper gepresst werden und mit einem Laserdurchstrahlschweißverfahren punktgenau entlang vorgegebener Konturen verschweißt werden. Insbesondere sind die Schweißkonturen durch die senkrechten Wandabschnitte, die auf die Folie gepresst werden, vorgegeben.

In einigen Fällen kann ein Teil oder alle Kanäle mit Folienstücken überdeckt werden, die genau der Größe des Kanals entsprechen. Produktionstechnisch ist es aber einfacher, größere Teile des Grundkörpers mit einer Folie zu belegen und entlang der Wandabschnittsverläufe der Flüssigkeitskanäle zu verschweißen. Auf diese Weise können die Flüssigkeitskanäle in einem Schweißprozess überdeckt werden.

Ebenso wie die Wandabschnitte der Flüssigkeitskanäle können die Wandabschnitte der Aufnahmeeinheit für das Zentrifugalpumpenmittel durch senkrechte Wandabschnitte gebildet werden, die senkrecht auf einer Basisplatte des Grundkörpers stehen. Insbesondere ist der Querschnitt der Aufnahmeeinheit im Wesentlichen kreisrund. Von der kreisrunden Form kann aber abgewichen werden, wenn durch Anschlüsse die geometrische Vorzugsform gestört werden muss. Insgesamt stellt die Aufnahmeeinheit eine zylindrische Form dar, in der bei Betrieb der Pumpeneinheit die zu verarbeitenden Flüssigkeit durch den Rotor beschleunigt wird und durch den Auslass in den ableitenden Kanal strömt.

Die Aufnahmeeinheit kann dabei mehr oder weniger hoch ausgestaltet sein. Dies hängt auch von der Geometrie des Rotors ab. Die Aufnahmeeinheit kann in Zusammenwirkung mit einer entsprechend kreisrunden Kappe überdeckt sein, so dass der Rotor in der Pumpeneinheit vollständig umschlossen wird. Die senkrechten Wandabschnitte der Aufnahmeeinheit, die Basisplatte die Überdeckung mit einer Kappe, Ein- und Auslass für Fluide und der Rotor bilden dabei die Pumpeneinheit.

Die Überdeckung kann auch durch eine Folie erfolgen, um Fertigungstoleranzen des Kassettengrundkörpers besser durch eine überdeckende Folie kompensieren zu können.

Der Rotorantrieb selbst ist von der Aufnahmeeinheit des Rotors getrennt und eine Komponente einer Maschinekonsole. Maschinenkonsole und Kassettenmodul sind so ausgeformt, dass sie komplementäre Oberflächenprofile aufweisen. Die Maschinenkonsole weißt entsprechend ein Oberflächenprofil auf, in das das Kassettenmodul eingelegt werden kann. Eine direkt gegenüberliegende Anordnung der entsprechend zugewandten Oberflächen von Maschinenkonsole und Kassettenmodul ist erforderlich, weil Interaktionen zwischen Maschinenkonsole und Kassettenmodul während des Betriebs der Einheit stattfinden. Solche Interaktionen entstehen z.B. durch Sensoren, die in der Maschinenkonsole untergebracht sind und die z.B. mechanisch, akustisch, thermisch, induktiv, optisch, magnetoresistiv oder elektrisch mit dem Kassettenmodul oder mit den darin im Betrieb des Kassettenmoduls geführten Flüssigkeiten in Wechselwirkung stehen. Dafür ist es notwendig, dass Sensoreinheiten und der zu analysierende Bereich des Kassettenmoduls geometrisch gegenüberliegen und für die Sensorsignalerfassung abgeglichen sind.

Ausgehend von einem Kassettenmodul der eingangs beschriebenen Art bestand daher weiterhin das Problem, eine Anordnung zu finden, bei der ein Kassettenmodul in eine entsprechende Konsole einer Dialysemaschine korrekt eingelegt werden kann, so dass die nötigen Interaktionen zwischen Dialysemaschine und Kassettenmodul funktionsgerecht ablaufen können. Dieses Problem wurde gelöst, indem auf der Maschinenkonsole ein Formteil angebracht ist, das mit einem entsprechend komplementär geformten Abschnitt auf dem Kassettenmodul eindeutig in Eingriff gebracht werden kann. "Eindeutig" ist hierbei so zu verstehen, dass es nur eine Positionierung von Kassette und Maschinenkonsole gibt, die zu einer formschlüssigen Anordnung führen kann. Das Formteil kann im Querschnitt vieleckig, z.B. drei- vier- oder fünfeckig, oval, asymmetrisch oder kreisförmig ausgeformt sein.

Die Kassette kann aber trotz korrekter Positionierung zur Maschinenkonsole eine gekippte oder geneigte Lage zur Maschinenkonsole einnehmen, so dass nicht alle Abschnitte von Kassette und Maschinenkonsole in direkte Interaktion treten können.

Zur Überprüfung einer korrekten Anordnung ist es daher weiterhin erforderlich, dass die Position von Kassette und Maschinenkonsole über eine weitere Verbindungseinheit verfügt, die eine Überprüfung zulässt, ob die Kassette komplett in einer korrekten Anordnung zu der Maschinenkonsole steht. Im Rahmen dieser Erfindung erfolgt diese Verbindung über einen Magneten. Dabei ist ein magnetisches Element konstruktiv in dem Kassettenmodul untergebracht. Ein entsprechendes magnetisches Gegenstück befindet sich in der Maschinenkonsole, so dass das Kassettenmodul durch anziehende magnetische Wechselwirkungen an der Maschinenkonsole gehalten wird. Die Position des Kassettenmoduls in der Anordnung mit der Maschinenkonsole wird über das erste Formteil der Maschinenkonsole und den Eingriff in das Kassettenmodul bereits teilweise vorgegeben. Die magnetische Verbindung sorgt dafür, dass die Kassette korrekt anliegt und nicht evtl. in einer fehlerhaften gekippten Lage an der Maschinenkonsole vorliegt.

Insbesondere ist vorgesehen, dass das magnetische Element auf einer ersten Seite des Kassettenmoduls untergebracht ist, die der Maschinenkonsole zugewandt ist. In einer weiteren Ausführung weist das Kassettenmodul hierfür eine Aufnahmeeinheit auf, in der sich das magnetische Element des Kassettenmoduls befindet. Alternativ handelt es sich bei dem magnetischen Element um einen magnetisches Zentrifugalpumpenmittel, also insbesondere um einen magnetischen Rotor einer Impellerpumpe. Der magnetische Antrieb auf der Maschinenkonsole ist so ausgestaltet, dass die Aufnahmeeinheit des Zentrifugalpumpenmittels in den magnetischen Antrieb eingepasst werden kann. Maschinenseitig kann über entsprechende Sensorik und Prozessoreinheit ein verändertes magnetisches Feld festgestellt werden, wenn sich die Aufnahmeeinheit mit dem magnetischen Rotor im Eingriff des Impellerantriebs befindet. Es kann so über eine Auswerteinheit im Prozessor und über die aufgenommenen Daten des veränderten magnetischen Feldes festgestellt werden, ob die Kassette in einer korrekten Anordnung mit der Maschinenkonsole steht. Dies kann zudem nur gewährleistet sein, wenn die Kassette auch im Eingriff mit dem Formteil der Maschinenkonsole steht.

Ein Kassettengrundkörper der eingangs genannten Art nach Anspruch 1 ist durch ein einstufiges Spritzgussverfahren herstellbar, das im Folgenden weiter ausgeführt wird.

Gemäß der geometrischen Anordnung von Flüssigkeitskanälen, Aufnahmeeinheit für das Zentrifugalpumpenmittel und Flüssigkeitsrampe am Auslasskanal der Aufnahmeeinheit auf einer ersten Seite und einer zweiten Seite des Grundkörpers kann der Grundkörper einstückig durch Spritzguss hergestellt werden.

Ein entsprechendes Spritzgussverfahren umfasst die Schritte des Bereitstellens eines Werkzeugs, das ein Profil aufweist, das einer ersten Seite des Grundkörpers komplementär ist. Wobei die erste Seite des Grundkörpers eine Seite ist, die Flüssigkeitskanäle enthält. Die Flüssigkeitskanäle sind dabei wie eingangs beschrieben aus senkrechten Wandabschnitten gebildet, die senkrecht auf einer Basisplatte des Grundkörpers angeordnet sind.

Die Basisplatte des Grundkörpers selbst weist Ausnehmungen auf, die eine Flüssigkeitsverbindung zwischen Flüssigkeitskanälen der ersten Seite und der Aufnahmeeinheit für das Zentrifugalpumpenmittel auf einer zweiten Seite des Grundkörpers ermöglicht. Die Ausnehmungen werden im Spritzgussprozess durch entsprechende Vorsprünge im Profil des ersten und/oder eines zweiten Werkzeuges geschaffen.

Unter einem Werkzeug ist im Zusammenhang von Spritzgussverfahren eine profilierte Halbform zu verstehen, deren Profil für die Aufnahme einer Polymerschmelze vorgesehen ist. Die Polymerschmelze wird dabei in den Hohlraum eingeströmt, der sich ergibt, wenn eine weitere Halbform auf die erste Halbform aufgesetzt wird.

Ein zweites Werkzeug gibt eine zweite profilierte Halbform vor und weist ein Profil auf, das in seiner Form zu einer zweiten Seite des Grundkörpers des Kassettenmoduls komplementär ist. Die zweite Seite ist dabei eine Seite, die die Aufnahmeeinheit für das Zentrifugalpumpenmittel aufweist. Die Aufnahmeeinheit wird wie die Flüssigkeitskanäle auf einer ersten Seite durch senkrechte Wandabschnitte gebildet, die senkrecht auf der Basisplatte des Grundkörpers angeordnet sind. Vorzugsweise ist die Aufnahmeeinheit durch eine zylindrische Form der Wandabschnitte aufgebaut. Entsprechend weist das zweite Werkzeug einen komplementären zylindrischen Bereich auf, der die Aufnahmeeinheit durch den Spritzgussprozess erzeugt.

Die Herstellung des Grundkörpers erfolgt, indem eine Polymerschmelze in den Hohlraum eingeleitet wird, der durch die beiden Werkzeughälften gebildet wird. Die Polymerschmelze verteilt sich in den zugänglichen Bereichen des Hohlraums und bildet dabei die Geometrie des Grundkörpers eines Kassettenmoduls aus. Der Grundkörper ist in diesem Prozess einstückig und beinhaltet Flüssigkeitskanäle, Aufnahmeeinheit für ein Zentrifugalpumpenmittel, Ausnehmungen in der Basisplatte, die eine Flüssigkeitsverbindung zwischen Flüssigkeitskanälen der ersten Seite des Grundkörpers und der zweiten Seite des Grundkörpers herstellen.

Nach Einfüllen der Polymerschmelze zwischen die Halbformen der Werkzeuge wird die Polymerschmelze unterhalb ihres Schmelzpunktes abgekühlt. Unter Schmelzpunkt ist in diesem Zusammenhang nicht allein ein thermodynamischer Schmelzpunkt zu verstehen. Im eigentlichen Sinn ist darunter zu verstehen, dass die zum Grundkörper erkaltete Polymerschmelze dem Werkzeug entnommen werden kann, ohne dass die Form des Grundkörpers beim Entformen kollabiert.

Bevorzugte Materialien für die Herstellung des Grundkörpers sind Polypropylene und Copolymere von Propylen und Ethylen. Daneben kann die Polymerschmelze auch thermoplastische elastomere Materialien enthalten, wie z.B. Polymere von Styrolblockcopolymere, z.B. auf Basis von Styrol, Ethylen und Buthylen Wiederhohlungseinheiten (SEBS) oder Styrol und Isopreneinheiten (SIS). Spritzgussteile, die aus diesen Materialien hergestellt werden können nach dem Prozessschritt des Erkaltens den Werkzeughälften entnommen werden und damit entformt werden.

Das Verfahren kann vorteilhaft weiter dazu genutzt werden, am Grundkörper eine Flüssigkeitsrampe zwischen der Aufnahmeeinheit für ein Zentrifugalpumpenmittel und der Ausnehmung in der Basisplatte zur Herstellung einer Flüssigkeitsverbindung zwischen der Aufnahmeeinheit einer zweiten Seite und einem Flüssigkeitskanal einer ersten Seite des Grundkörpers herzustellen. Die Flüssigkeitsrampe wird durch einen schräg ansteigenden Boden in dem Wandabschnitt der Aufnahmeeinheit gebildet, der von einem der Basisplatte entfernten Bereich zu der Ausnehmung in der Basisplatte im Wandabschnittbereich der Aufnahmeeinheit angeordnet ist. Die Profilierung der ersten und zweiten Werkzeughälften ist dementsprechend komplementär ausgestaltet.

Insbesondere ist die Verwendung eines Kassettenmoduls der eingangs beschriebenen Art entsprechend einem der Ansprüche 1 bis 13 in der Dialyse vorgesehen. Das für diese Verwendung besonders geeignete Kassettenmodul zeichnet sich, wie eingangs beschrieben, durch die Anordnung von Flüssigkeitskanälen auf einer Seite des Grundkörpers und der Aufnahmeeinheit für ein Zentrifugalpumpenmittel auf einer zweiten Seite des Grundkörpers aus, wobei Flüssigkeitskanäle und Aufnahmeeinheit durch Ausnehmungen in der Basisplatte des Grundkörpers flüssigkeitsverbindend ausgestaltet sind. In dieser Anordnung kann der Grundkörper einstückig durch das beschriebene Spritzgussverfahren hergestellt werden. Die flüssigkeitsführenden Komponenten der Kassette müssen daher nicht aus einzelnen Teilen zusammengesetzt werden. Die Verbindungen der Komponenten zu einer zusammengesetzten Einheit müssten durch Verbindungstechniken, wie z.B. Klebetechniken oder Schweißtechniken, erfolgen.

Neben dem erhöhten Fertigungsaufwand würde dies auch für die in Dialysebehandlungen zu verarbeitenden Flüssigkeiten, z.B. Blut oder Dialysierflüssigkeiten, ein gesteigertes Schädigungsrisiko bedeuten. Zum einen können fertigungsbedingt dann Toträume oder Kantenvorsprünge entstehen, die zu Blutkoagulation oder Hämolyse führen können. Ebenso weist ein Kassettenmodul mit zusammengesetzter Grundplatte immer ein höheres Risiko durch Partikelkontamination auf, als einstückig hergestellte Kassettenmodule.

### Kurze Beschreibung der Zeichnungen

Figur 1 zeigt ein im Stand der Technik bekannte Zentrifugalpumpe
Figur 2 zeigt einen Ausschnitt aus einem Grundkörper eines erfindungsgemäßen Kassettemoduls aus einer Perspektive, die eine erste Seite mit Flüssigkeitskanälen sichtbar macht.
Figur 3 zeigt den Ausschnitt aus dem Grundkörper eines erfindungsgemäßen Kassettenmoduls der Figur 2 aus einer weiteren Perspektive, die eine zweite Seite mit einer Aufnahmeeinheit für ein Zentrifugalpumpenmittel sichtbar macht.
Figur 4 zeigt eine Querschnittsansicht aus einem Ausschnitt eines Grundkörpers nach den Figuren 2 und 3.
Figur 5 zeigt eine Querschnittsansicht einer zu den Figuren 2 und 3 alternativen Ausführung eines Grundkörpers eines Kassettenmoduls.
Figur 6 zeigt einen Ausschnitt aus einem erfindungsgemäßen Kassettenmoduls in einer Perspektive auf eine erste Seite des Kassettenmoduls.
Figur 7 zeigt eine Querschnittsansicht des Ausschnitts aus einem Kassettenmodul nach Figur 6.
Figur 8 zeigt einen Ausschnitt aus dem Grundkörper einer alternativen Ausführung eines erfindungsgemäßen Kassettenmoduls in einer Perspektive, die eine erste Seite sichtbar macht.
Figur 9 zeigt eine weitere alternative Ausführung aus dem Grundkörper eines Kassettenmoduls in einer Perspektive, die eine erste Seite des Kassettenmoduls sichtbar macht, auf der die Flüssigkeitskanäle durch eine Folie verdeckt sind. Zusätzlich ist eine Querschnittsansicht der weiteren alternativen Ausführung gezeigt.
Figur 10 zeigt eine Querschnittsansicht einer Anordnung aus Maschinenkonsole und einem Abschnitt eines erfindungsgemäßen Kassettenmoduls.

### Detaillierte Beschreibung anhand der Zeichnung

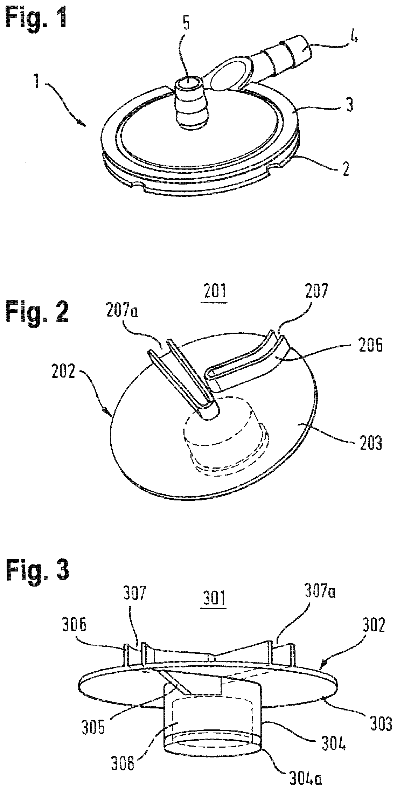

Figur 1 zeigt ein Gehäuse einer Zentrifugalpumpe 1, wie sie im Stand der Technik bekannt ist. Die Zentrifugalpumpe besteht aus einem oberen Gehäuseteil 3 und einem unteren Gehäuseteil 2. Ein Einlassport 5 ist senkrecht auf dem oberen Gehäuseteil 3 angebracht. Ein Auslassport 4 ist tangential am unteren Gehäuseteil 2 angebracht. Zuströmende Flüssigkeit wird im Betrieb der Pumpeneinheit über den Einlassport 5 entlang der Rotationsachse des Rotors 6 in das Gehäuse 1 eingeleitet, und durch den Auslassport 4 wieder ausgeleitet. Der Auslass der Flüssigkeit erfolgt tangential zum Rotor 6, weil hier die Geschwindigkeit der Flüssigkeit am größten ist.

Figur 2 zeigt in einer perspektivischen Darstellung einen Ausschnitt aus einem nicht näher dargestellten Kassettenmodul 201 mit einem Grundkörper 202 umfassend eine Basisplatte 203. Gezeigt ist eine erste Seite des Grundkörpers 202 mit der Anordnung von Flüssigkeitskanälen 207, 207a. Die Flüssigkeitskanäle werden durch senkrechte Seitenwände 206 gebildet. Verdeckt in dieser Ansicht befindet sich auf einer zweiten Seite des Grundkörpers 202 und der Basisplatte 203 die Aufnahmeeinheit für den Rotor zur Förderung von Flüssigkeiten.

Figur 3 zeigt den Ausschnitt aus Figur 2 in einer geänderten perspektivischen Darstellung. Darin ist zu sehen, dass ein Ausschnitt aus dem Kassettenmodul 301 einen Grundkörper 302 mit einer Basisplatte 303 beinhaltet. Zu sehen ist in dieser Darstellung eine zweite Seite des Grundköpers 302 mit einer Aufnahmeeinheit 304 für einen Rotor als Zentrifugalpumpenmittel 308 (verdeckt). Die Aufnahmeneinheit 304 besteht aus einem zylindrischen Gehäuse und steht in Flüssigkeitsverbindung mit den Flüssigkeitskanälen 307, 307a, gebildet durch senkrechte Seitenwände 306 der ersten Seite des Grundkörpers. Die Flüssigkeitsverbindung eines tangentialen Auslasses der Aufnahmeeinheit 304 liegt direkt benachbart zu einer Flüssigkeitsrampe 305, so dass ausströmende Flüssigkeit von der Aufnahmeeinheit der zweiten Seite des Grundköpers in den Flüssigkeitskanal 307 der zweiten Seite gefördert wird. Das Zentrifugalpumpenmittel 308 wird weiter von einer überdeckenden Kappe 304a in der Aufnahmeeinheit gehalten.

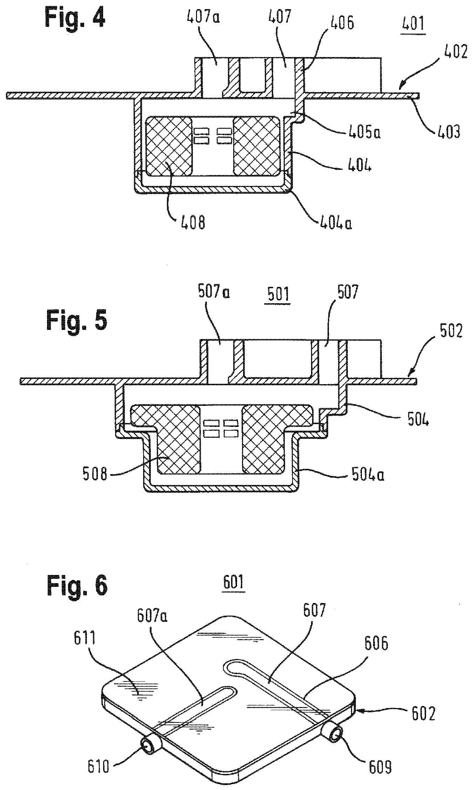

Figur 4 zeigt einen zur Basisplatte 203 aus Figur 2 gezeigten senkrechten Schnitt durch den Grundkörper 202. Darin ist der entsprechende Ausschnitt 401 aus der Kassette gezeigt, bestehend aus einem Grundkörper 402 und der Basisplatte 403 des Grundkörpers. Als Grundkörper sind generell alle konstruktiven zusammenhängende Details zu verstehen, die in der Darstellung zu einer integralen und einstückigen Ausbildung beitragen. Als integrale Bestandteile des Grundkörpers 402 und generell der Grundkörper der anderen Abbildungen sind zu verstehen:
die Aufnahmeinheit 404 für den Rotor,
der tangentiale Auslass aus der Aufnahmeeinheit 405a, der zu der in dieser Figur nicht gezeigten Flüssigkeitsrampe führt,
die senkrechten Seitenwände 406, die die Flüssigkeitskanäle 407, 407a bilden
die Basisplatte 403.

In der Aufnahmeeinheit ist in geschnittener Darstellung der Rotor 408 zu erkennen, der durch die überdeckende Kappe 404a in der Aufnahmeeinheit 404 gehalten wird. Im Betrieb der Pumpeneinheit wird zuströmende Flüssigkeit durch den einlassenden Kanal 407a in die Aufnahmeeinheit eingeleitet, durch den Impeller 408 beschleunigt und durch den Auslass in den Flüssigkeitskanal 407 geleitet.

In einer alternativen Ausführung zu Figur 4 wird auf die Aufnahmeeinheit 404 an der Basisplatte verzichtet. Entsprechend ist die Kappe 404a so ausgestaltet, dass die Aufnahmeeinheit in die Kappe integriert ist. Insbesondere ist dann auch die Flüssigkeitsrampe in die Kappe integriert.

Figur 5 zeigt eine weitere Ausführung eines Ausschnitts 501 eines Kassettenmoduls mit dem Grundkörper 502 und den Flüssigkeitskanälen 507, 507a. Sie unterscheidet sich gegenüber Figur 4 durch eine geänderte Geometrie des Rotors 508, Aufnahmeeinheit 504 und überdeckender Kappe 504a. Bestimmte Anforderungen an die Pumpleistung der Pumpeneinheit, z. B. Förderdruck und Flussrate können alternative Geometrien des Rotors notwendig machen. Aufnahmeeinheit und überdeckende Kappe müssen geometrisch entsprechend angepasst werden. Im vorliegenden Fall ist schematisch ein Rotor gezeigt, dessen Durchmesser auf der Seite, die der Basisplatte 503 zugewandt ist, einen größeren Durchmesser aufweist, als auf der Seite, die der überdeckenden Kappe zugewandt ist. Die Aufnahmeeinheit 504 ist von den geometrischen Abmessungen entsprechend auf den Durchmesser des Rotors angepasst. Die Kappe 504a ist geometrisch dem Sprung des Querschnittsdurchmessers des Rotors angepasst.

Figur 6 zeigt eine perspektivische Ansicht eines Ausschnitts 601 eines erfindungsgemäßen Kassettenmoduls, wobei die Seitenwände 606 der Flüssigkeitskanäle (verdeckt) mit einer überdeckenden Folie 611 verschweißt sind. Auf der Folie schematisch eingezeichnet sind die Konturen der Schweißzonen, die den Konturen der senkrechten Seitenwände 606 der Flüssigkeitskanäle 607 und 607a entsprechen. Die Folie 611 wird durch die Schweißung fest mit dem Grundkörper 602 über die Seitenwände 606 verbunden. 609 und 610 zeigen Ports der im Betrieb der Pumpeneinheit ableitenden und zuleitenden Flüssigkeitskanäle. Die Folie 611 überdeckt dabei mehrere Flüssigkeitskanäle und in der gezeigten Darstellung sogar den gesamten Ausschnitt 601 des Kassettenkörpers.

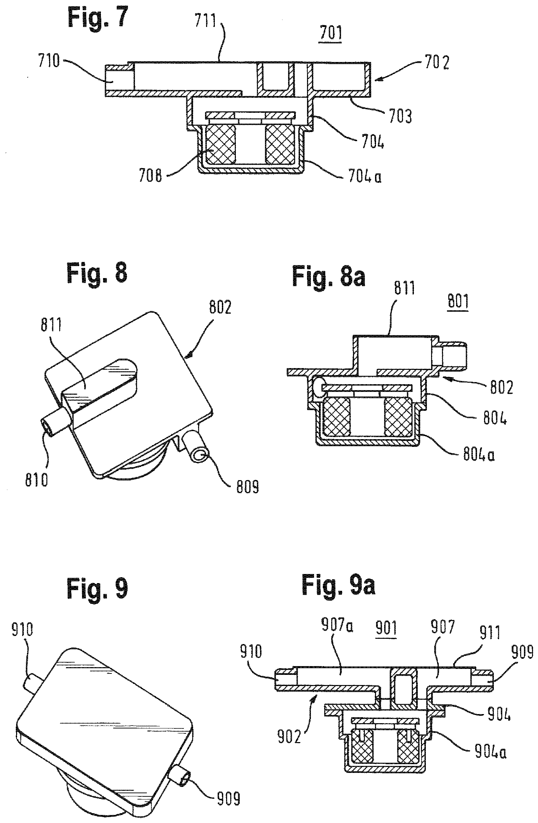

Figur 7 zeigt eine Querschnittsansicht eines Ausschnitts 701 aus einem Kassettenmodul entsprechend Figur 6. Die Figur zeigt eine alternative Ausgestaltung der Pumpeneinheit bestehend aus Aufnahmeeinheit 704, Kappe 704a und Rotor 708. Port 710 entspricht dem Port 610 des Zuleitungskanals aus Figur 6. Folie 711 entspricht der überdeckenden Folie 611 aus Figur 6. 702 bezeichnet den Grundkörper, 703 die Basisplatte, 707a und 707 die Flüssigkeitskanäle.

Figur 8 zeigt ein weitere Ausgestaltung eines Ausschnitts 801 aus einem Kassettenmodul mit Blickrichtung auf eine erste Seite des Grundkörpers 802. Ports 810 und 809 bilden Zugänge zu den zuleitenden und ableitenden Flüssigkeitskanälen, die mit der Pumpeneinheit in Verbindung stehen. Die Figur zeigt eine Ausführung bei der nur der gezeigte Flüssigkeitskanal des Kassettenmoduls mit einem Folienstück 811 überdeckt und mit den senkrechten Seitenwänden des Flüssigkeitskanals verschweißt wurde.

Figur 8a zeigt eine Schnittansicht eines senkrechten Schnitts durch den Ausschnitt des Kassettenmoduls von Figur 8. Die Figur zeigt den Grundkörper, der integral mit der Aufnahmeeinheit 804 verbunden ist. Es ist zu erkennen, dass der zuleitende Flüssigkeitskanal 807 auf der ersten Seite des Grundkörpers angeordnet ist, wohingegen der ableitende Flüssigkeitskanal mit dem ableitenden Port 809 auf der zweiten Seite des Grundkörpers 802 angeordnet ist. Nach wie vor bilden die Flüssigkeitskanäle der ersten Seite und die Aufnahmeeinheit und Flüssigkeitskanäle der zweiten Seite des Grundkörpers eine integrale Einheit, die im einstufigen Spritzgussverfahren einstückig hergestellt werden kann. Die

Anordnung des ableitenden Flüssigkeitskanal auf der zweiten Seite des Grundkörpers kann bestimmten strömungsmechanischen Anforderung Rechnung tragen. Anders als in Ausführungen mit einer Flüssigkeitsrampe, z.B. Figur 3, geht bei einer Ausführung wie in Figur 8a gezeigt weniger Strömungsenergie der zu fördernden Flüssigkeit verloren, da sie nicht über die schräge Rampe auf die erste Seite des Grundkörpers umgeleitet werden muss. Die Rampe kann bei bestimmten Anwendungen den Förderdruck und Strömungsrate der Flüssigkeit ungünstig herabsetzen. Andererseits kann es in bestimmten Ausführungen vorteilhaft sein, Strukturierungen auf der zweiten Seite des Kassettenmoduls, die im Betrieb der Behandlungsmaschine zugewandt sind und mit dieser im Eingriff steht, so gering wie möglich zu halten. Dieser Vorteil würde sich bei Ausführungen gemäß den Abbildungen 2 bis 7 ergeben, bei denen die Flüssigkeitskanäle auf der ersten Seite des Grundkörpers angeordnet sind.

Figur 9 zeigt eine weitere Ausgestaltung eines Abschnitts 901 eines Kassettenmoduls, wobei, wie in Figur 9a zu sehen ist, die Aufnahmeeinheit für das Zentrifugalpumpenmittel nicht integral mit dem Grundkörper 902 verbunden ist. Die Aufnahmeeinheit ist als Kopplungsplatte 904 zwischen der Kappe 904a und dem Grundkörper 903 angeordnet und bildet ein separates Bauteil, das durch Verschweißung oder Verklebung mit dem Grundkörper verbunden werden kann. Der Rotor 908 wird von der Kappe 904a in dem Pumpengehäuse (gebildet aus Kopplungsplatte 904 und Kappe 904a) eingefasst. Zu- und ableitende Ports und Flüssigkeitskanäle 907, 907a, 910 und 909 sind integral mit dem Grundkörper verbunden. Die Kopplungsplatte 904 weist entsprechende Durchgänge für die Zu- und Ableitung von zu fördernden Flüssigkeiten auf. Die Kappe 904a kann mit einer in Figur 9 nicht dargestellten Flüssigkeitsrampe ausgestattet sein, wie sie in der Ausführung zu Figur 3 beschrieben wurde. Die Flüssigkeitsrampe erfüllt entsprechend die Funktionen der Flüssigkeitsrampe 305 aus Figur 3.

Die Flüssigkeitskanäle des Grundkörpers sind durch eine Folie 911 überdeckt. Diese Anordnung kann insbesondere dann von Bedeutung sein, wenn die Aufnahmeeinheit aus bestimmten Gründen aus einem anderen Material als der Grundkörper bestehen muss. Für bestimmte Ausführungen kann es notwendig sein, Vibrationen, die durch den Betrieb der Pumpeneinheit auf die Grundplatte übertragen werden, zu eliminieren. Dies kann erreicht werden, indem ein dämpfendes Material für die Kopplungsplatte 904 verwendet wird. Allgemein ist es vorteilhaft, wenn der Grundkörper aus einem Polypropylen Copolymer (random PP), einem Polykarbonat (PC) oder einem Polyeyterterephtalat (PET) in einem Spritzgussverfahren hergestellt wird. Die Kopplungsplatte 904 kann dann thermoplastische elastomere Kunststoffmaterialien, wie z.B. SEBS, Ethylen-Butylen Kautschuke, oder andere elastomere Materialien enthalten, die mechanische Vibrationen des Rotors dämpfen und nur vermindert auf die Grundplatte weiterleiten. Es kann so vermieden werden, dass durch Vibrationen andere Funktionseinrichtungen im Betrieb empfindlich gestört werden könnte.

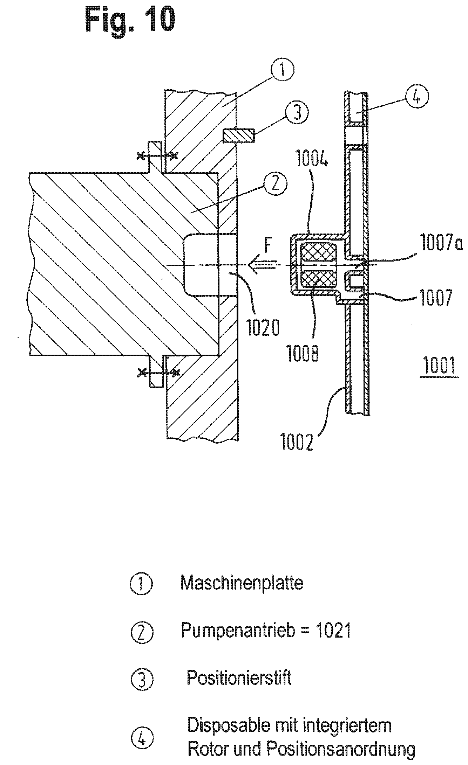

Figur 10 zeigt einen Ausschnitt 1001 aus einem Kassettenmodul mit einem integrierten Rotor 1008, wie es in den Ausführungen der vorangehenden Abbildungen beschrieben wurde. Die Figur zeigt das über die Grundplatte 1002 hervorstehende Pumpengehäuse 1004, das eine Aufnahmeeinheit des Rotors der voran beschriebenen Ausführungen beinhalten kann. Das Pumpengehäuse 1004 mit Rotor 1008 ist so ausgestaltet, dass es von einer Vertiefung 1020 des Pumpenantriebs 1021 aufgenommen werden kann. Der Antrieb erzeugt die entsprechenden Magnetfelder, die den Rotor 1008 in Bewegung versetzen.

Daneben zeigt die Figur 10 ein Formteil 3 an der Maschinenplatte, das Teil der Maschinekonsole ist, die dafür vorgesehen ist mit dem Kassettenmodul im Eingriff zu stehen. Das Formteil ist in Figur 10 beispielhaft als ein Positionsstift ausgebildet. Zusammen mit der Vertiefung 1020 des Rotorantriebs kann die Kassette positionsgenau an die Maschinenkonsole angeordnet und zudem über ein nicht gezeigtes Sensorsystem in der Rotorantriebseinheit verifiziert werden.

## Patentansprüche

1. Kassettenmodul für die Verarbeitung von Blut und therapeutischen Flüssigkeiten in der extrakorporalen Blutbehandlung, der Dialyse oder der Infusionstechnik umfassend:
einen Grundkörper (202, 302, 402, 502, 602, 702, 802, 902, 1002), mit einer ersten Seite und einer der ersten Seite gegenüberliegenden zweiten Seite,
auf dem Grundkörper integrierte Flüssigkeitskanäle (207, 207a, 307, 307a, 407, 407a, 507, 507a, 607, 607a, 707, 707a, 907, 907a, 1007, 1007a), und
eine Aufnahmeeinheit (304, 404, 504, 704, 804, 904, 1004) zur Aufnahme eines Zentrifugalpumpenmittels (308, 408, 508, 708, 1008), zur Förderung von Flüssigkeiten, wobei
der Grundkörper eine Basisplatte aufweist, die die erste Seite und die zweite Seite des Grundkörpers definiert,
wenigstens ein Teil der Flüssigkeitskanäle auf der ersten Seite angeordnet und die Aufnahmeeinheit für das Zentrifugalpumpenmittel auf der zweiten Seite angeordnet ist,
die Aufnahmeeinheit für das Zentrifugalpumpenmittel im Betrieb in Flüssigkeitsverbindung mit den Flüssigkeitskanälen der ersten Seite steht,
die Flüssigkeitskanäle (207, 207a, 307, 307a, 407, 407a, 507, 507a, 607, 607a, 707, 707a, 907, 907a, 1007, 1007a) teilweise durch Seitenwände (206, 306, 406, 606) gebildet werden, die auf dem Grundkörper senkrecht zu der Erstreckungsfläche der Basisplatte angebracht sind und die Flüssigkeitskanäle seitlich begrenzen, wobei die Flüssigkeitskanäle (207, 207a, 307, 307a, 407, 407a, 507, 507a, 607, 607a, 707, 707a, 907, 907a, 1007, 1007a) auf der ersten oder der zweiten Seite durch eine Folie (611, 711, 811, 911) überdeckt und mit den seitlichen begrenzenden senkrecht stehenden Seitenwänden (206, 306, 406, 606) wenigstens teilweise verbunden sind, so dass die Flüssigkeitskanäle durch die Basisplatte, den senkrechten Seitenwänden und der Folie begrenzt sind.

2. Kassettenmodul nach Anspruch 1, **dadurch gekennzeichnet, dass** die Flüssigkeitskanäle (207, 207a, 307, 307a, 407, 407a, 507, 507a, 607, 707, 707a, 907, 907a, 1007, 1007a) auf der ersten Seite Zu- und Ableitung der Fluidströme zum Zentrifugalpumpenmittel bilden.

3. Kassettenmodul nach einem der vorangehenden Ansprüche, **dadurch gekennzeichnet, dass** die Aufnahmeeinheit für das Zentrifugalpumpenmittel (304, 404, 504, 704, 804, 904, 1004) der zweiten Seite einen Auslass für Flüssigkeiten aufweist und dass dieser Auslass mit einer Flüssigkeitsrampe (305) verbunden ist, die die Aufnahmeeinheit auf der zweiten Seite mit einem Flüssigkeitskanal der ersten Seite (207, 307, 407, 507, 607, 707, 907, 1007) in Flüssigkeitsverbindung bringt.

4. Kassettenmodul nach Anspruch 1, **dadurch gekennzeichnet, dass** die Aufnahmeeinheit (304, 404, 504, 704, 804, 904, 1004) auf der zweiten Seite einen Auslass aufweist, der mit einem Flüssigkeitskanal der zweiten Seite in Flüssigkeitsverbindung steht.

5. Kassettenmodul nach Anspruch 4, **dadurch gekennzeichnet, dass** ein Flüssigkeitskanal zum Zuleiten eines Fluids zum Zentrifugalpumpenmittel auf der ersten Seite angeordnet ist und ein Flüssigkeitskanal zum Ableiten eines Fluids auf der eine zweiten Seite angeordnet ist.

6. Kassettenmodul nach einem oder mehreren der vorangehenden Ansprüche, **dadurch gekennzeichnet, dass** die Aufnahmeeinheit (304, 404, 504, 704, 804, 1004) integral mit dem Grundkörper verbunden ist.

7. Kassettenmodul nach Anspruch 6, **dadurch gekennzeichnet, dass** der Grundkörper (202, 302, 402, 502, 602, 702, 802, 902, 1002) mit der Aufnahmeeinheit (304, 404, 504, 704, 804, 904, 1004) für das Zentrifugalpumpenmittel (308, 408, 508, 708, 1008) und den Flüssigkeitskanälen (207, 207a, 307, 307a, 407, 407a, 507, 507a, 607, 607a, 707, 707a, 907, 907a, 1007, 1007a) ein Spritzgussteil darstellt.

8. Kassettenmodul nach einem der vorhergehenden Ansprüche 1-5, **dadurch gekennzeichnet, dass** der Grundkörper (902) mit Flüssigkeitskanälen (907, 907a) und die Aufnahmeeinheit (904) für das Zentrifugalpumpenmittel zwei separate Teile darstellen, die miteinander verbunden sind.

9. Kassettenmodul nach einem oder mehreren der vorangehenden Ansprüche, **dadurch gekennzeichnet, dass** die Aufnahmeeinheit (304, 404, 504, 704, 804, 904, 1004) für das Zentrifugalpumpenmittel (308, 408, 508, 708, 1008) auf der zweiten Seite teilweise durch Seitenwände gebildet werden, die auf dem Grundkörper senkrecht zu der Erstreckungsfläche des Grundkörpers angebracht sind und die Aufnahmeeinheit seitlich begrenzen.

10. Kassettenmodul nach einem der vorhergehenden Ansprüche, **dadurch gekennzeichnet, dass** mehrere Flüssigkeitskanäle (207, 207a, 307, 307a, 407, 407a, 507, 507a, 607, 607a, 707, 707a, 907, 907a, 1007, 1007a) auf einer Seite des Grundkörpers durch eine Folie (611, 711, 811, 911) überdeckt und mit den seitlichen begrenzenden senkrecht stehenden Seitenwänden (206, 306, 406, 606) wenigstens teilweise verbunden sind, so dass die Flüssigkeitskanäle durch die Basisplatte, den senkrechten Seitenwänden und der Folie begrenzt sind, wobei vorzugsweise
die Aufnahmeeinheit (304, 404, 504, 704, 804, 904, 1004) des Zentrifugalpumpenmittels (308, 408, 508, 708, 1008) einen Aufnahmetopf bildet, in dem der Rotor des Zentrifugalpumpenmittels aufgenommen ist, und die Seitenwände mit einer Folie überdeckt und verbunden sind und den Aufnahmetopf begrenzen.

11. Anordnung aus einem Kassettenmodul nach einem der Ansprüche 1 bis 10 und einer Maschinenkonsole einer Maschine für die extrakorporale Blutbehandlung oder der Dialyse, wobei
das Kassettenmodul den Grundkörper (1002) aufweist, der mit der Aufnahmeeinheit (1004) für ein magnetisches Zentrifugalpumpenmittel (1008) auf der zweiten Seite des Grundkörpers, die in der Anordnung der Maschinenkonsole zugeordnet ist, und den Flüssigkeitskanälen (1007, 1007a) auf der ersten Seite des Kassettenmoduls, die in der Anordnung von der Maschinenkonsole abgewandt ist, versehen ist,
die Anordnung ferner ein Formteil (3) an der Maschinenkonsole, das mit einem komplementären Formteil der Kassette im formschlüssigen Eingriff steht, und einen Impellerantrieb (2), der in die Maschinenkonsole integriert ist, aufweist, und
die Kassette in der Anordnung im formschlüssigen und magnetischen Eingriff mit dem Zentrifugalpumpenmittel des Kassettenmoduls steht.

## Claims

1. A cassette module for processing blood and therapeutic fluids in an extracorporeal blood treatment, dialysis or infusion technology, comprising:
a base body (202, 302, 402, 502, 602, 702, 802, 902, 1002) having a first side and a second side opposite the first side,
fluid channels (207, 207a, 307, 307a, 407, 407a, 507, 507a, 607, 607a, 707, 707a, 907, 907a, 1007, 1007a) integrated on the base body, and
a receiving unit (304, 404, 504, 704, 804, 904, 1004) for receiving a centrifugal pump means (308, 408, 508, 708, 1008) for delivering fluids, wherein
the base body has a base plate which defines the first side and the second side of the base body,
at least some of the fluid channels are arranged on the first side and the receiving unit for the centrifugal pump means is arranged on the second side,
the receiving unit for the centrifugal pump means is in fluid communication with the fluid channels on the first side during operation,
the fluid channels (207, 207a, 307, 307a, 407, 407a, 507, 507a, 607, 607a, 707, 707a, 907, 907a, 1007, 1007a) are partially formed by side walls (206, 306, 406, 606) which are mounted on the base body at a right angle to the extension surface of the base plate and bound the fluid channels at the sides,
with the fluid channels (207, 207a, 307, 307a, 407, 407a, 507, 507a, 607, 607a, 707, 707a, 907, 907a, 1007, 1007a) on the first side or the second side being covered by a film (611, 711, 811, 911) and being at least partially connected to the lateral bounding perpendicular side walls (206, 306, 406, 606) bounding the channels at the sides so that the fluid channels are bounded by the base plate, the perpendicular side walls and the film.

2. The cassette module according to claim 1, **characterized in that** the fluid channels (207, 207a, 307, 307a, 407, 407a, 507, 507a, 607, 707, 707a, 907, 907a, 1007, 1007a) on the first side form inlet and outlet lines for the fluid flows to the centrifugal pump means.

3. The cassette module according to one of the preceding claims, **characterized in that** the receiving unit (304, 404, 504, 704, 804, 904, 1004) for the centrifugal pump means on the second side has an outlet for fluids, and **in that** this outlet is connected to a fluid ramp (305) which brings the receiving unit on the second side into fluid communication with a fluid channel on the first side (207, 307, 407, 507, 607, 707, 907, 1007).

4. The cassette module according to claim 1, **characterized in that** the receiving unit (304, 404, 504, 704, 804, 904, 1004) has an outlet on the second side, said outlet being in fluid communication with a fluid channel on the second side.

5. The cassette module according to claim 4, **characterized in that** a fluid channel for conducting a fluid to the centrifugal pump means is arranged on the first side, and a fluid channel for removing a fluid is arranged on the one second side.

6. The cassette module according to one or more of the preceding claims, **characterized in that** the receiving unit (304, 404, 504, 704, 804, 1004) is integrally connected to the base body.

7. The cassette module according to claim 6, **characterized in that** the base body (202, 302, 402, 502, 602, 702, 802, 902, 1002) together with the receiving unit (304, 404, 504, 704, 804, 904, 1004) for the centrifugal pump means (308, 408, 508, 708, 1008) and the fluid channels (207, 207a, 307, 307a, 407, 407a, 507, 507a, 607, 607a, 707, 707a, 907, 907a, 1007, 1007a) is an injection-molded part.

8. The cassette module according to one of the preceding claims 1 to 5, **characterized in that** the base body (902) with the fluid channels (907, 907a) and the receiving unit (904) for the centrifugal pump means are two separate parts which are connected to one another.

9. The cassette module according to one or more of the preceding claims, **characterized in that** the receiving unit (304, 404, 504, 704, 804, 904, 1004) for the centrifugal pump means (308, 408, 508, 708, 1008) on the second side is formed in part by side walls which are mounted on the base body at a right angle to the extension surface of the base body and bound the receiving unit at the sides.

10. The cassette module according to one of the preceding claims, **characterized in that** multiple fluid channels (207, 207a, 307, 307a, 407, 407a, 507, 507a, 607, 607a, 707, 707a, 907, 907a, 1007, 1007a) on one side of the base body are covered by a film (611, 711, 811, 911) and are at least partially connected to the lateral bounding perpendicular side walls (206, 306, 406, 606) so that the fluid channels are bounded by the base plate, the perpendicular side walls and the film, wherein preferably
the receiving unit (304, 404, 504, 704, 804, 904, 1004) of the centrifugal pump means (308, 408, 508, 708, 1008) forms a receiving pot in which the rotor of the centrifugal pump means is accommodated, and the perpendicular side walls are covered with and connected to a film and bound the receiving pot.

11. An arrangement of a cassette module according to one of claims 1 to 10 and of a machine console of a machine for extracorporeal blood treatment or dialysis, wherein
the cassette module has the base body (1002) which is provided with the receiving unit (1004) for a magnetic centrifugal pump means (1008) on the second side of the base body which side is assigned to the machine console in the arrangement, and which is provided with the fluid channels (1007, 1007a) on the first side of the cassette module which side faces away from the machine console in the arrangement,
the arrangement furthermore has a molded part (3) on the machine console which is in form-fitting engagement with a complementary molded part of the cassette and an impeller drive (2) which is integrated into the machine console, and
the cassette in the arrangement is in form-fitting and magnetic engagement with the centrifugal pump means of the cassette module.

## Revendications

1. Module à cassette destiné au traitement du sang et de liquides thérapeutiques dans le traitement extracorporel du sang, la dialyse ou la technique de perfusion, comprenant :
un corps de base (202, 302, 402, 502, 602, 702, 802, 902, 1002), doté d'un premier côté et d'un second côté opposé au premier côté,
des canaux à liquide (207, 207a, 307, 307a, 407, 407a, 507, 507a, 607, 607a, 707, 707a, 907, 907a, 1007, 1007a) intégrés sur le corps de base, et
une unité de réception (304, 404, 504, 704, 804, 904, 1004) destinée à recevoir un moyen formant pompe centrifuge (308, 408, 508, 708, 1008) pour le transport de liquides,
le corps de base comportant une plaque de base, qui définit le premier côté et le second côté du corps de base,
au moins une partie des canaux à liquide étant disposée sur le premier côté et l'unité de réception pour le moyen formant pompe centrifuge étant disposé sur le second côté,
l'unité de réception pour le moyen formant pompe centrifuge étant, pendant le fonctionnement, en liaison fluidique avec les canaux à liquide du premier côté,
les canaux à liquide (207, 207a, 307, 307a, 407, 407a, 507, 507a, 607, 607a, 707, 707a, 907, 907a, 1007, 1007a) étant formés en partie par des parois latérales (206, 306, 406, 606), qui sont placées sur le corps de base perpendiculairement à la surface d'extension de la plaque de base et qui limitent latéralement les canaux à liquide,
les canaux à liquide (207, 207a, 307, 307a, 407, 407a, 507, 507a, 607, 607a, 707, 707a, 907, 907a, 1007, 1007a) étant recouverts sur le premier ou le second côté par une feuille (611, 711, 811, 911) et étant reliés au moins en partie aux parois latérales (206, 306, 406, 606) perpendiculaires réalisant une limitation latérale, de telle sorte que les canaux à liquide sont limités par la plaque de base, les parois latérales perpendiculaires et la feuille.

2. Module à cassette selon la revendication 1, **caractérisé en ce que** les canaux à liquide (207, 207a, 307, 307a, 407, 407a, 507, 507a, 607, 707, 707a, 907, 907a, 1007, 1007a) sur le premier côté forment des conduites d'alimentation et d'évacuation des flux de fluide pour le moyen formant pompe centrifuge.

3. Module à cassette selon l'une des revendications précédentes, **caractérisé en ce que** l'unité de réception pour le moyen formant pompe centrifuge (304, 404, 504, 704, 804, 904, 1004) du second côté comporte une sortie pour liquides et **en ce que** cette sortie est reliée à une rampe à liquide (305), qui met l'unité de réception sur le second côté en liaison fluidique avec un canal à liquide du premier côté (207, 307, 407, 507, 607, 707, 907, 1007).

4. Module à cassette selon la revendication 1, **caractérisé en ce que** l'unité de réception (304, 404, 504, 704, 804, 904, 1004) sur le second côté comporte une sortie, qui est en liaison fluidique avec un canal à liquide du second côté.

5. Module à cassette selon la revendication 4, **caractérisé en ce qu'**un canal à liquide est disposé sur le premier côté pour l'alimentation d'un fluide dans le moyen formant pompe centrifuge et un canal à liquide est disposé sur le second côté pour évacuer un fluide.

6. Module à cassette selon une ou plusieurs des revendications précédentes, **caractérisé en ce que** l'unité de réception (304, 404, 504, 704, 804, 1004) est reliée au corps de base de manière intégrée.

7. Module à cassette selon la revendication 6, **caractérisé en ce que** le corps de base (202, 302, 402, 502, 602, 702, 802, 902, 1002) représente avec l'unité de réception (304, 404, 504, 704, 804, 904, 1004) pour le moyen formant pompe centrifuge (308, 408, 508, 708, 1008) et les canaux à liquide (207, 207a, 307, 307a, 407, 407a, 507, 507a, 607, 607a, 707, 707a, 907, 907a, 1007, 1007a) une pièce moulée par injection.

8. Module à cassette selon l'une des revendications précédentes 1 à 5, **caractérisé en ce que** le corps de base (902) avec les canaux à liquide (907, 907a) et l'unité de réception (904) pour le moyen formant pompe centrifuge représentent deux pièces séparées, qui sont reliées l'une à l'autre.

9. Module à cassette selon une ou plusieurs des revendications précédentes, **caractérisé en ce que** l'unité de réception (304, 404, 504, 704, 804, 904, 1004) pour le moyen formant pompe centrifuge (308, 408, 508, 708, 1008) sur le second côté est formée en partie par des parois latérales, qui sont placées sur le corps de base perpendiculairement à la surface d'extension du corps de base et qui limitent latéralement l'unité de réception.

10. Module à cassette selon l'une des revendications précédentes, **caractérisé en ce que** plusieurs canaux à liquide (207, 207a, 307, 307a, 407, 407a, 507, 507a, 607, 607a, 707, 707a, 907, 907a, 1007, 1007a) sont recouverts sur un côté du corps de base par une feuille (611, 711, 811, 911) et au moins en partie reliés aux parois latérales (206, 306, 406, 606) perpendiculaires réalisant une limitation latérale, de telle sorte que les canaux à liquide sont limités par la plaque de base, les parois latérales perpendiculaires et la feuille, l'unité de réception (304, 404, 504, 704, 804, 904, 1004) du moyen formant pompe centrifuge (308, 408, 508, 708, 1008) formant de préférence un pot de réception, dans lequel le rotor du moyen formant pompe centrifuge est reçu, et les parois latérales étant recouvertes d'une feuille et reliées à celle-ci et limitant le pot de réception.

11. Agencement constitué d'un module à cassette selon l'une des revendications 1 à 10 et d'un panneau de machine d'une machine pour le traitement extracorporel du sang ou la dialyse,
le module à cassette comportant le corps de base (1002), qui est pourvu de l'unité de réception (1004) pour un moyen formant pompe centrifuge (1008) magnétique sur le second côté du corps de base, qui est associé au panneau de machine dans l'agencement, et des canaux à liquide (1007, 1007a) sur le premier côté du module à cassette, qui est orienté dans la direction opposée au panneau de machine dans l'agencement,
l'agencement comportant en outre une pièce moulée (3) sur le panneau de machine, qui est en prise par complémentarité de forme avec une pièce moulée complémentaire de la cassette, et un entraînement d'impulseur (2), qui est intégré dans le panneau de machine, et
la cassette étant, dans l'agencement, en prise magnétique et par complémentarité de forme avec le moyen formant pompe centrifuge du module à cassette.
